(19) Europäisches Patentamt
European Patent Office
Office européen des brevets

(11) Publication number: **0 410 351 B1**

(12) **EUROPEAN PATENT SPECIFICATION**

(45) Date of publication of patent specification: **07.12.94**

(51) Int. Cl.$^5$: **C08K 5/14**, C08L 27/12, C07C 409/16

(21) Application number: **90114081.4**

(22) Date of filing: **23.07.90**

(54) **Curable mixtures of fluoroelastomers containing bromine or iodine and of organic peroxides.**

(30) Priority: **24.07.89 IT 2127789**

(43) Date of publication of application:
**30.01.91 Bulletin 91/05**

(45) Publication of the grant of the patent:
**07.12.94 Bulletin 94/49**

(84) Designated Contracting States:
**AT BE DE ES FR GB NL SE**

(56) References cited:
**DE-A- 1 469 998**
**DE-A- 3 715 210**
**DE-A- 3 925 743**

(73) Proprietor: **AUSIMONT S.p.A.**
**31, Foro Buonaparte**
**I-20100 Milano (IT)**

(72) Inventor: **Chiodini, Graziella,**
**Via Pastore, 30**
**Saronno, Varese (IT)**
Inventor: **Lagostina, Attilio,**
**8, Via Clemente**
**Spinetta Marengo, Alessandria (IT)**
Inventor: **Merenda, Michele,**
**5, Via Alessandria**
**Frugarolo, Alessandria (IT)**
Inventor: **Minutillo, Anna,**
**8, Via Etiopia**
**Milano (IT)**
Inventor: **Montessoro, Ezio,**
**11, Via San Audina**
**Spinetta Marengo, Alessandria (IT)**

(74) Representative: **Weinhold, Peter, Dr. et al**
**Patentanwälte Dipl.-Ing. G. Dannenberg**
**Dr. P. Weinhold**
**Dr. D. Gudel**
**Dipl.-Ing. S. Schubert**
**Dr. P. Barz**
**Siegfriedstrasse 8**
**D-80803 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

The present invention relates to mixtures based on fluoroelastomers containing Br and/or I, which mixtures comprise particular organic peroxides and exhibit, during curing, a low emission of toxic vapors of methyl bromide and/or iodide.

It is widely known to cure fluoroelastomers containing, as cure sites, bromine or iodine atoms whithin the polymeric chain and/or at the end of said chain with organic peroxides.

In such fluoroelastomers, bromine or iodine atoms are introduced into the elastomer macromolecule by using brominated or iodinated comonomers such as, in particular, fluorobrominated olefins, brominated or iodinated fluorovinyl ethers or by using, during the polymerization process, brominated or iodinated compounds as chain transfer agents.

In the peroxide cure of said elastomers there are utilized, in general, organic peroxides of the aliphatic type, saturated or unsaturated, such as, e.g., 2,5-dimethyl-2,5-di(tert-butylperoxy)hexyne-3 and 2,5-dimethyl-2,5-di(tert-butylperoxy)hexane, which give rise, in the curing process, to the formation of methyl radicals. These radicals, by combination with bromine or iodine contained in the fluoroelastomer, form methyl bromide or methyl iodide, which are highly toxic volatile compounds and, in the case of the iodide, are also cancerogenic.

Therefore, while the peroxide curing of fluoroelastomers containing Br or I permits to obtain cured articles having improved stability to steam and other aggressive agents, as compared with articles obtained through ionic curing, it is apparent, on the other hand, that said peroxide curing involves a serious hazard for the health of the operators entrusted with the processing of said elastomers.

Furthermore, it is not possible to avoid said harmful emissions merely by using peroxides which do not release at all or release only small amounts of methyl radicals during their decomposition, because said peroxides will not cure at all or cure only weakly said fluoroelastomers. This becomes evident, for example, when using an organic perketal of formula

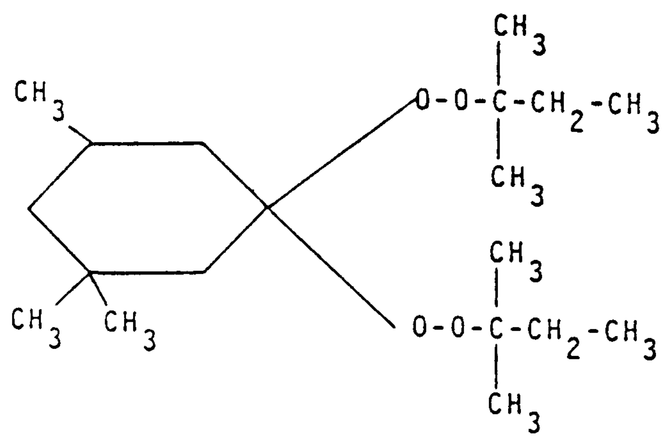

which, by decomposition, provides prevailingly ethyl radicals and only small amounts of methyl radicals, but which is not capable of curing fluoroelastomers containing bromine or iodine.

Thus, it is an object of the present invention to substantially reduce or even eliminate the harmful emissions of methyl bromide and/or methyl iodide which occur during the radical curing of fluoroelastomers containing Br and/or I without, however, affecting the good rheological properties of the vulcanizate.

It has now surprisingly been found that it is possible to significantly reduce the formation of said toxic products by using particular organic peroxides, not only without jeopardizing the cure trend and results, but - conversely - even improving the cure rate and the crosslinking yield. Moreover, it has been found that it is possible to further reduce or eliminate the emission of methyl bromide or iodide if, in combination with said particular peroxides, small amounts of organic additives, specified hereinafter, are utilized.

Consequently, object of the present invention are curable mixtures based on fluoroelastomers containing bromine and/or iodine atoms in the polymeric chain, which comprise organic peroxides as radical crosslinking agents and are characterized in that the peroxides are selected from monoperoxides of formula (I):

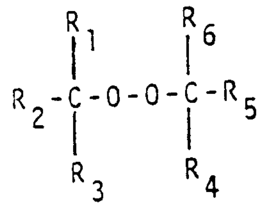

(I)

wherein:

$R_1$ and $R_6$, identical or different from each other, are $C_{2-4}$-alkyl groups;

$R_3$ and $R_4$, identical or different from each other, are $C_{1-4}$-alkyl groups;

$R_2$ and $R_5$, identical or different from each other, are $C_{1-4}$-alkyl groups and phenyl groups optionally substituted by $C_{1-4}$-alkyl groups;

and bisperoxides of formula (II):

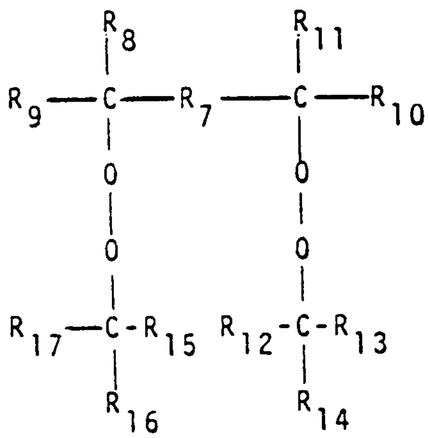

(II)

wherein:

$R_7$ is $C_{1-6}$-alkylene, $C_{2-6}$-alkenylene, $C_{2-6}$-alkynylene or phenylene;

$R_8$, $R_9$, $R_{10}$ and $R_{11}$, identical or different from one another, are $C_{1-3}$-alkyl groups;

$R_{13}$ and $R_{15}$, identical or different from each other, are $C_{2-4}$ alkyl groups;

$R_{12}$, $R_{14}$, $R_{16}$ and $R_{17}$, the same or different from one another, are $C_{1-4}$-alkyl groups;

provided that when $R_7$ is phenylene, $R_8$ and $R_{11}$, the same or different from each other, are $C_{2-4}$-alkyl groups.

Examples of the just mentioned alkyl groups are methyl, ethyl, n- and i-propyl. Examples of alkylene groups are methylene, ethylene, propylene and butylene.

A further object of the present invention are curable compositions based on fluoroelastomers containing Br and/or I, which comprise, besides the peroxides of formula (I) and/or (II), also small amounts, generally ranging from 0.1 to 1 part by weight per 100 parts of rubber (p.h.r.), of benzothiazole and derivatives thereof, preferably 2-mercapto-benzothiazole and its salts, in particular zinc salts, mercapto-benzothiazole disulphide (MBTS), morpholine-2-benzothiazole-sulphenamide, and/or 0.1-2 p.h.r. of N-phenyl maleimide (NPM) or derivatives thereof.

By the additional use of small amounts of benzothiazole or its derivatives it is possible to achieve a complete suppression, during curing, of the emission of methyl bromide and/or iodide, while retaining the good characteristics of the vulcanizate prepared by using the peroxides of formula (I) and/or (II). An additional advantage is that the scorch time is increased thereby, resulting in a higher processing safety without affecting the crosslinking rate.

Examples of fluoroelastomers containing bromine and/or iodine atoms as cure sites are copolymers of $CH_2$ = $CF_2$ and $CF_3$-CF= = $CF_2$ and, optionally , $C_2F_4$, and the copolymers of $C_2F_4$ and perfluorovinylethers, said copolymers containing small amounts of bromine or iodine, introduced by copolymerizing a small amount of brominated or iodinated monomer in the form of brominated (iodinated) olefins, perfluorobromoalkyl-perfluoro-vinylethers, or also by the use of chain transfer agents, e.g. bromoalkyl or iodoalkyl compounds.

Some of the peroxides of general formulae (I) or (II) are known while others are new compounds, namely:
bis(1,1-diethylpropyl) peroxide,
bis(1-ethyl-1-methylpropyl) peroxide and
1,1-diethylpropyl-1-ethyl-1-methylpropyl-peroxide.

Said new compounds form a further object of the present invention.

The amounts of peroxides of formulae (I) or (II) to be employed in the mixtures according to the present invention generally range from 1 part to 6 parts by weight per 100 parts of elastomer (p.h.r.), and in particular from 1.5 to 3 p.h.r.

Said peroxides, besides substantially reducing the emission of toxic methyl bromide and/or iodide by at least 90% and, in some cases, by more than 95%, offer the following advantages:

- increase by 20% to 150% in the crosslinking rate at 180 °C,
- in some cases, increase in the crosslinking yield at 180 °C,
- improvement in the release of the article from the mold.

In the preparation of mixtures, the peroxides can also be utilized carried on inert fillers, such as e.g. calcium carbonate or silica or mixtures thereof, which makes their use easier and permits to avoid the problems caused by their volatility.

The mixtures based on fluoroelastomers containing bromine and/or iodine according to the present invention usually comprise, besides organic peroxide(s) and, optionally, benzothiazole, derivatives thereof and/or N-phenylmaleimide, the conventional ingredients utilized in curable mixtures, such as carbon black and other reinforcing fillers, peroxide curing coagents (for example triallyl isocyanurate), metal oxides and hydroxides (for example PbO, ZnO), and processing aids.

The following examples are given to merely illustrate but not to limit the present invention.

Examples 1-15

Tests were carried out to determine the $CH_3Br$ emissions of mixtures containing the peroxides according to the invention and in some cases also mercaptobenzothiazole disulphide (MBTS) or N-phenylmaleimide (NPM), in comparison with mixtures containing a conventional peroxide (2,5-dimethyl-2,5-di-tert-butylperoxy-hexane (Luperco® 101 XL)).

The tested mixtures were all based on terpolymer P.1, i.e. a terpolymer composed of 66.2% by moles of $CH_2=CF_2$, 18.2% by moles of $C_3F_6$, 15.2% by moles of $C_2F_4$ and containing bromoperfluorovinylether in amounts equal to 0.65% by weight of bromine. The components of the mixtures, besides the peroxides and the additives of the types and in the amounts indicated in Table 1, were the following:

- 4 p.h.r. of triallyl isocyanurate (TAIC) at 75% on an inert filler,
- 3 p.h.r. of PbO,
- 30 p.h.r. of carbon black MT.

The $CH_3Br$ emissions during the curing step were determined according to the following method:
20 g of a fine particle mixture (polymer + fillers) were treated for 30 minutes at 180 °C in a closed reactor and at a pressure of 0.5 kg/cm$^2$ abs. in an $N_2$ atmosphere.

At the end, the whole mixture was cooled to 40-50 °C and, as an internal standard, 1 ml of A 114 ($C_2Cl_2F_4$, b.p. = 4 °C) was added.

The gas phase was mixed and then analyzed in a gas-chromatograph.

Column: POROPAK® Q

Temperature: 100 °C.

The cure trend was evaluated by determining the Δ torque (MH-ML) by means of an oscillating disk rheometer (ODR) (Monsanto type), according to standard ASTM D 2084/81, with "arc ± 3".

Table 1 shows the results,where the $CH_3Br$ emissions are expressed as percent reduction of the standard emission of a mixture containing the conventional peroxide Luperco® 101 XL (Example 1).

## TABLE 1

| EX. | PEROXIDE | phr | ADDITIVE phr | TORQUE MH - ML | MAX | REDUCTION IN % OF $CH_3Br$ EMISSION |
|---|---|---|---|---|---|---|
| 1 | Luperco®101 XL<br>* carried for 45% | 3 | - | 67 | 0,79 | - |
| 2 | (1) | 1,6 | - | 65 | 0,71 | 95 |
| 3 | (1) | 1,8 | - | 66 | 0,74 | 94 |
| 4 | (1) | 2,0 | - | 74 | 1,05 | 96 |
| 5 | (1) | 1,8 | MBTS 0,25 | 71 | 0,87 | 100 |
| 6 | (1) | 1,8 | NPM 1,5 | 75 | 1,01 | 98 |
| 7 | (1) - * carried for 40% | 4,5 | - | 69 | 0,78 | 96 |
| 8 | (1) - * carried for 40% | 4,5 | MBTS 0,25 | 66 | 0,66 | 100 |
| 9 | (2) | 1,3 | - | 50 | 1,08 | 95 |
| 10 | (3) - * carried for 40% | 5,1 | - | 66 | 1,15 | 95 |
| 11 | (3) - * carried for 40% | 6,8 | - | 68 | 1,24 | 95 |
| 12 | (3) - * carried for 40% | 8,5 | - | 68 | 1,37 | 94 |
| 13 | (3) - * carried for 40% | 8,5 | MBTS 0,25 | 73 | 1,60 | 100 |
| 14 | (4) | 1,5 | - | 70 | 0,95 | 95 |
| 15 | (5) | 2,6 | - | 60 | 1,3 | 98 |

* carried on $CaCO_3/SiO_2$ 1:1

(1) = bis(1,1-dimethylpropyl)peroxide

(2) = bis(1,1-diethylpropyl)peroxide

(3) = bis(1-ethyl-1-methylpropyl)peroxide

(4) = 2,5-dimethyl-2,5-di-tert-amyl-peroxy-hexane

(5) = 1,1-diethylpropyl, 1-ethyl-1-methylpropyl-peroxide

## Example 16

Curing tests in a mold were carried out to determine the reduction, under actual conditions, of the $CH_3Br$ emissions by subjecting to gas-chromatographic analysis, under the conditions indicated in the preceding examples, an air sample withdrawn from above the molded article immediately after the release of the vulcanizate from the mold.

The elastomers utilized in the mixtures were the P.1 defined in the preceding examples and P.40, which is a terpolymer composed of 54.1% by moles of $CH_2=CF_2$, 22.2% by moles of $C_3F_6$ and 22.9% by moles of $C_2F_4$ and containing bromoperfluoroethylvinylether in an amount equal to 0.6% by weight of bromine. Both the mixtures and the resulting cured products were examined. The following Tables show the characteristics in comparison with standard mixtures which contain Luperco® 101 XL as peroxide. In particular, Table 2 relates to the compositions of the tested mixtures and their characteristics. Table 3 shows the percent reduction of the $CH_3Br$ emissions and some characteristic values, which are indicative of the cure trend.
Table 4 gives the characteristics of the cured material.

TABLE 2

| COMPOSITION OF THE MIX (ASTM D.3182-82) | | | | | | | ASTM D. 1646-82 | | |
|---|---|---|---|---|---|---|---|---|---|
| | | | | | | | 135° C | | 121° C |
| Mix No. | polymer | peroxide | phr | TAIC | PbO | carbon black | MOONEY MV | SCORCH t 15 | MOONEY ML (1'+10') |
| 1 | P. 1 | LUPERCO® 101 XL 45% | 3,0 | 4 | 3 | 30 | 48 | 13'36" | 103 |
| 2 | P. 1 | PURE (1) | 1,8 | 4 | 3 | 30 | 48 | 10'55" | 103 |
| 3 | P. 40 | LUPERCO® 101 XL 45% | 3,0 | 4 | 3 | 30 | 12 | 26'30" | 33 |
| 4 | P. 40 | PURE (1) | 1,8 | 4 | 3 | 30 | 17 | 20'30" | 42 |

(1) = bis(1,1-dimethylpropyl)peroxide.

TABLE 3

| POLYMER | MIX No. (from tab. 2) | CURING CHARACTERISTICS (ASTM 02084-81 - ODR, 180°C; arc +/-3) ML (lbf.in) | MH (lbf.in) | ts 2 (s) | t'50 (s) | t'90 (s) | V max (lbf.in/s) | Δ TORQUE MH-ML | REDUCTION IN % OF $CH_3Br$ EMISSION |
|---|---|---|---|---|---|---|---|---|---|
| P. 1 | 1 | 22 | 80 | 72 | 126 | 360 | 0,58 | 58 | - |
| P. 1 | 2 | 22 | 86 | 66 | 123 | 294 | 0,70 | 64 | 92% |
| P.40 | 3 | 4 | 51 | 78 | - | 399 | 0,40 | 47 | - |
| P.40 | 4 | 6 | 55 | 75 | - | 270 | 0,60 | 49 | 96% |

TABLE 4

| POLYMER | MIX No. (from tab.2) | MECHANICAL PROPERTIES – after press-cure (170°C + 10') – ASTM D.412-83 – Tensile propert. (MPa) M100 | (MPa) M200 | (MPa) T.S. | (%) E.B. | ASTM D.2240-8 – Hardness (points) H-Shore A | after post-cure (250°C + 24 h) – ASTM D.412-83 – Tensile properties (MPa) M100 | (MPa) M200 | (MPa) T.S. | (%) E.B. | ASTM D2240-81 Hardness (points) H-Shore A | ASTM D1414-78 (*) C.S. (%) O-R 214 |
|---|---|---|---|---|---|---|---|---|---|---|---|---|
| P. 1 | 1 | 3.7 | 9.1 | 12 | 284 | 72 | 5.6 | 15.9 | 18.2 | 233 | 75 | 33 |
| P. 1 | 2 | 4.4 | 11.7 | 14 | 257 | 73 | 6.0 | 17.7 | 21.2 | 231 | 73 | 25 |
| P.40 | 3 | 2.4 | 5.1 | 6.4 | 383 | 66 | 4.0 | 10.0 | 13.4 | 262 | 70 | 44 |
| P.40 | 4 | 3.0 | 6.3 | 7.4 | 339 | 68 | 4.4 | 11.2 | 14.3 | 252 | 71 | 45 |

(*) C.S. = compression set at 200°C for 70 hours.

Example 17 (comparison test)

Curing tests were carried out at 180 °C in a mold, using a mixture having the composition indicated in examples 1 to 15, except that the peroxide used was a perketal which, by decomposition formed prevailingly ethyl radicals and some methyl radicals ($CH_3$-$CH_2$•/$CH_3$• ratio = 15:1) and had the formula:

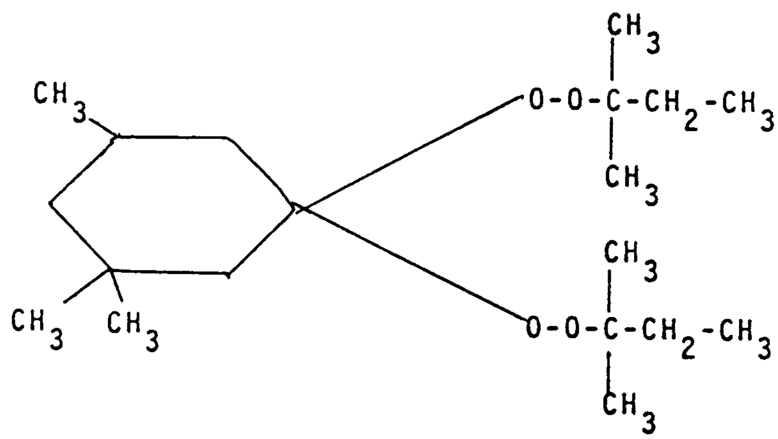

Said peroxide, which was utilized in the mixtures in an amount of 1.7 p.h.r., led to a viscosity increase ( Δ torque) of only 7 points, as compared to 67 points obtained by using Luperco® 101 XL (see Table 1), which indicates that it did not initiate the cure of the fluoroelastomers at 170°-180°C.

<u>Example 18</u>

Synthesis of bis(1,1-dimethylpropyl)peroxide of formula:

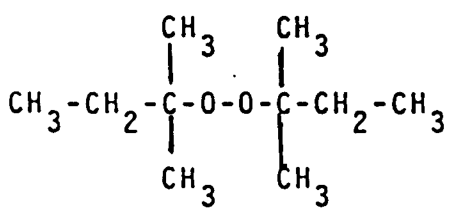

A 1 l 4-necked flask equipped with stirrer, thermometer and efficient cooler was charged with 400 g of 2-methyl-2-butanol (4.54 moles).

After having brought the temperature to 40°C, 115.8 g of $H_2O_2$ at 70% (2.383 moles) and 453.4 g of $H_2SO_4$ at 70% (3.236 moles) were simultaneously introduced over 60 minutes.

The reaction was stopped after 6 hours by discontinuing the stirring. After separation, 318.4 g of crude product were obtained (86.5% iodometric titre as peroxide), which was then purified by repeated washings with equal portions of NaOH at 10%. The washed product was then purified by means of rectification under vacuum, thereby obtaining 250 g of product at 99.5% of gas-chromatographic titre.

The obtained product was identified by means of NMR, gas-mass and iodometric analyses.

<u>Example 19</u>

Synthesis of bis(1,1-diethylpropyl)peroxide of formula:

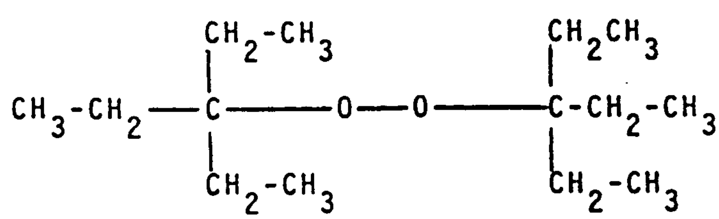

First step

In a 500 ml flask equipped with stirrer and cooling system, 1 mole of triethylcarbinol was reacted with 2 moles of $H_2O_2$ at 70% in the presence of 1 mole of $H_2SO_4$ and in 1.3 moles of hexane (solvent), at a temperature of 20 ° C for 2 to 4 hours.

Upon completion of the reaction the organic phase was separated from the aqueous phase and neutralization was carried out with 20 g of an aqueous NaOH solution at 1%.

Second step

To the organic phase of the first step there was then added 1 mole of triethylcarbinol, and to the resulting mixture, after evaporation of the solvent at 0 ° C under vacuum, 2 moles of para-toluenesulphonic acid (aqueous solution at 70%) were added. Thereafter the reaction mixture was kept at 35 ° C for 24 hours.

From the reaction mixture-the peroxide was separated by decantation of the organic phase and subsequently it was purified by means of repeated washings with an aqueous NaOH solution at 10%.

After filtration and removal under vacuum of the light fractions, a residue was obtained, which residue consisted of bis(1,1-diethylpropyl)peroxide, which was identified by NMR, iodometric and gas-mass analyses.

Example 20

Synthesis of bis(1-ethyl-1-methylpropyl)peroxide of formula:

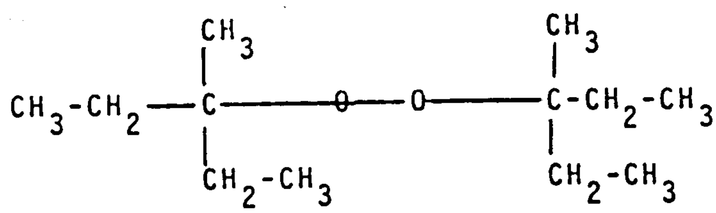

First step

A 0.5 l 4-necked flask equipped with stirrer, thermometer and efficient cooler was charged with 100 g of 3-methyl-3-pentanol (0.979 moles). After having brought the temperature to 30 ° C, 95.1 g of $H_2O_2$ at 70% (1.957 moles) and 137.1 g of $H_2SO_4$ at 70% (0.979 moles) were simultaneously introduced over 30 minutes. The reaction was stopped after 3 hours.

After separation of the phases, 112.8 g of 1-methyl-1-ethylpropyl hydroperoxide at 99% of iodometric titre were obtained.

Second step

A l liter flask was charged with 112.8 g of hydroperoxide and 100 g of 3-methyl-3-pentanol (0.979 moles). The whole mixture was brought to 40 ° C and 394.5 g of p-toluenesulphonic acid at 70% (1.603 moles) were introduced within 20 minutes.

The reaction was stopped after 18 hours and 120.5 g of a crude product were obtained. The crude product was purified by means of repeated washings with NaOH at 20% and then was rectified under vacuum, affording 95 g of peroxide at 96% of iodometric titre, identified by NMR and gas-mass analyses.

Example 21

Synthesis of 1,1-diethylpropyl, 1-ethyl-1-methylpropylperoxide of formula:

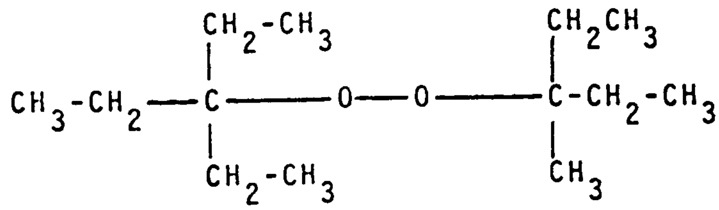

The procedure of example 19 was followed, with the exception that in the second step diethylcarbinol was utilized instead of triethylcarbinol.

The peroxide was characterized by means of NMR, iodometric and gas-mass analyses.

Example 22

Synthesis of 2,5-dimethyl-2,5-di-tert-amyl-peroxy-hexane of formula:

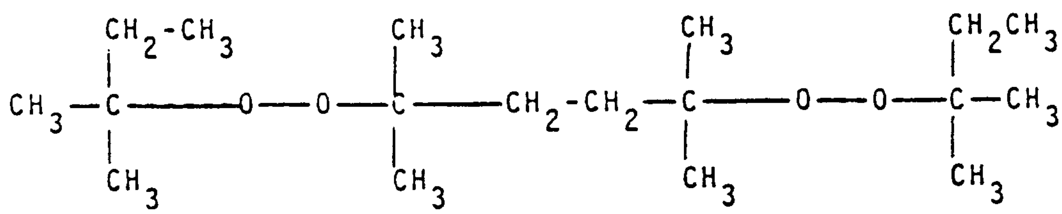

A 1 l 4-necked flask equipped with stirrer, thermometer and efficient cooler was charged with 176.3 g of 2-methyl-2-butanol (2.0 moles) and 445.7 g of 2,5-dimethyl-2,5-bis (hydroperoxy)hexane at 80% (2 moles). While maintaining the temperature at 30 °C, 280.3 g of $H_2SO_4$ at 70% (2.0 moles) were then introduced within 30 minutes.

The reaction was stopped after 6 hours by discontinuing stirring and separating the two phases which had formed. The resulting crude product (400 g) was repeatedly washed with aqueous NaOH at 10% and demineralized water in equal amounts.

After rectification under vacuum (residual pressure of 11 mm Hg), there were obtained 350 g of product at 90% (gas-chromatographic titre) which, by NMR, gas-mass and iodometric analyses, was identified as 2,5-dimethyl-2,5-bis(tert-amyl-peroxy)hexane.

## Claims

**1.** Curable mixtures based on fluoroelastomers which contain bromine and/or iodine in the polymeric chain and comprising organic peroxides as radical crosslinking agents, characterized in that the peroxides are selected from monoperoxides of general formula (I):

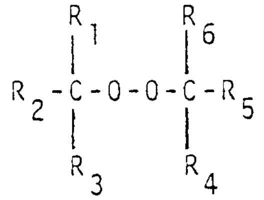

(I)

wherein:

$R_1$ and $R_6$, the same or different from each other, are $C_{2-4}$-alkyl groups;

$R_3$ and $R_4$, the same or different from each other, are $C_{1-4}$-alkyl groups;

$R_2$ and $R_5$, the same or different from each other, are $C_{1-4}$-alkyl groups, or phenyl groups optionally substituted with $C_{1-4}$-alkyl groups;

and bisperoxides of general formula (II):

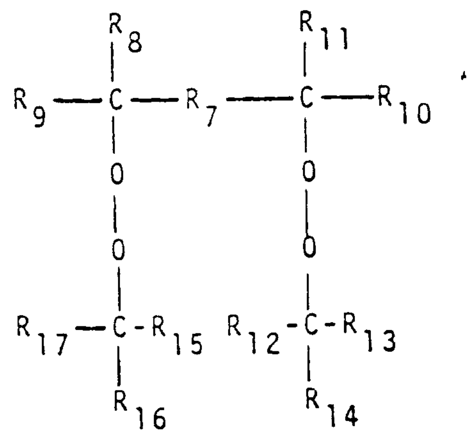

(II)

wherein:

$R_7$ is $C_{1-6}$-alkylene, $C_{2-6}$-alkenylene, $C_{2-6}$-alkynylene or phenylene;

$R_8$, $R_9$, $R_{10}$ and $R_{11}$, the same or different from one another, are $C_{1-3}$-alkyl groups;

$R_{13}$ and $R_{15}$, the same or different from each other, are $C_{2-4}$-alkyl groups;

$R_{12}$, $R_{14}$, $R_{16}$ and $R_{17}$, the same or different from one another, are $C_{1-4}$-alkyl groups;

provided that when $R_7$ is phenylene, $R_8$ and $R_{11}$, the same or different from each other, are $C_{2-4}$-alkyl groups.

**2.** Mixtures according to claim 1, wherein the peroxide is selected from bis(1,1-dimethylpropyl)peroxide, bis(1,1-diethylpropyl)peroxide, bis(1-ethyl-1-methylpropyl)peroxide, 1,1-diethylpropyl, 1-ethyl-1-methylpropyl-peroxide, 2,5-dimethyl-2,5-di-tert-amylperoxy-hexane and mixtures thereof.

**3.** Mixtures according to anyone of claims 1 and 2, furthermore containing, preferably in amounts of from 0.1 to 1 part by weight, benzothiazole or derivatives thereof, particularly mercaptobenzothiazole disulphide.

**4.** Mixtures according to anyone of claims 1 to 3, containing, preferably in amounts of from 0.1 to 2 parts by weight, N-phenyl maleimide.

**5.** Bis(1,1-diethylpropyl)peroxide.

**6.** Bis(1-ethyl-1-methylpropyl)peroxide.

**7.** 1,1-Diethylpropyl, 1-ethyl-1-methylpropyl-peroxide.

## Patentansprüche

**1.** Härtbare Mischungen auf der Basis von Fluorelastomeren, die Brom und/oder Jod in der polymeren Kette enthalten und organische Peroxide als Radikal-Vernetzungsmittel umfassen, dadurch gekennzeichnet, daß die Peroxide aus Monoperoxiden der allgemeinen Formel (I)

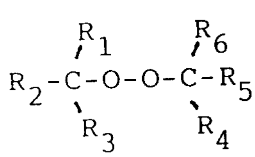

(I)

worin

$R_1$ und $R_6$, die gleich oder voneinander verschieden sind, $C_2$-$C_4$-Alkylgruppen bedeuten,

$R_3$ und $R_4$, die gleich oder voneinander verschieden sind, $C_1$-$C_4$-Alkylgruppen bedeuten,

$R_2$ und $R_5$, die gleich oder voneinander verschieden sind, $C_1$-$C_4$-Alkylgruppen oder Phenylgruppen, die gegebenenfalls mit $C_1$-$C_4$-Alkylgruppen substituiert sind, bedeuten;

und Bisperoxiden der allgemeinen Formel (II) ausgewählt sind:

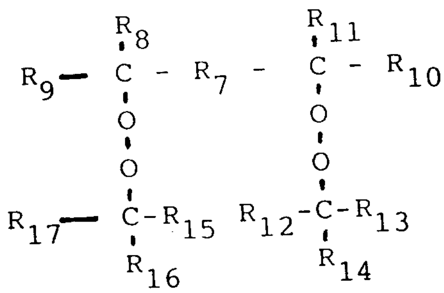

(II)

worin

$R_7$ $C_1$-$C_6$-Alkylen, $C_2$-$C_6$-Alkenylen, $C_2$-$C_6$-Alkinylen oder Phenylen bedeutet,

$R_8$, $R_9$, $R_{10}$ und $R_{11}$, die gleich oder voneinander verschieden sind, $C_1$-$C_3$-Alkylgruppen bedeuten,

$R_{13}$ und $R_{15}$, die gleich oder voneinander verschieden sind, $C_2$-$C_4$-Alylgruppen bedeuten,

$R_{12}$ $R_{14}$, $R_{16}$ und $R_{17}$, die gleich oder voneinander verschieden sind, $C_1$-$C_4$-Alkylgruppen bedeuten,

mit der Maßgabe, daß, wenn $R_7$ Phenylen ist, $R_8$ und $R_{11}$, die gleich oder voneinander verschieden sind, $C_2$-$C_4$-Alkylgruppen bedeuten.

2. Mischungen gemäß Anspruch 1, worin das Peroxid aus Bis(1,1-dimethylpropyl)peroxid, Bis(1,1-diethylpropyl)peroxid, Bis(1-ethyl-1-methylpropyl)peroxid, 1,1-Diethylpropyl, 1-ethyl-1-methylpropylperoxid, 2,5-Dimethyl-2,5-di-t-amylperoxy-hexan und Mischungen derselben ausgewählt ist.

3. Mischungen gemäß irgendeinem der Ansprüche 1 und 2, die ferner, vorzugsweise in Mengen von 0,1 bis 1 Gew.-Teil, Benzothiazol oder dessen Derivate, insbesondere Mercaptobenzothiazoldisulfid, enthalten.

4. Mischungen gemäß irgendeinem der Ansprüche 1 bis 3, die, vorzugsweise in Mengen von 0,1 bis 2 Gew.-Teilen, N-Phenylmaleimid enthalten.

5. Bis(1,1-diethylpropyl)peroxid.

6. Bis(1-ethyl-1-methylpropyl)peroxid.

7. 1,1-Diethylpropyl-1-ethyl-1-methylpropyl-peroxid.

## Revendications

1. Mélanges durcissables à base de fluoroélastomères qui contiennent du brome et/ou de l'iode dans la chaîne polymère, et comprenant des peroxydes organiques comme agents de réticulation radicalaire, caractérisés par le fait que les peroxydes sont choisis parmi :

- les monoperoxydes de formule générale (I) :

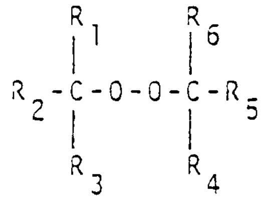

(I)

dans laquelle :

- $R_1$ et $R_6$, identiques ou différents l'un de l'autre, représentent des groupes alkyle en $C_{2-4}$ ;
- $R_3$ et $R_4$, identiques ou différents l'un de l'autre, représentent des groupes alkyle en $C_{1-4}$ ;
- $R_2$ et $R_5$, identiques ou différents l'un de l'autre, représentent des groupes alkyle en $C_{1-4}$, ou des groupes phényle facultativement substitués par des groupes alkyle en $C_{1-4}$ ; et

- les bisperoxydes de formule générale (II) :

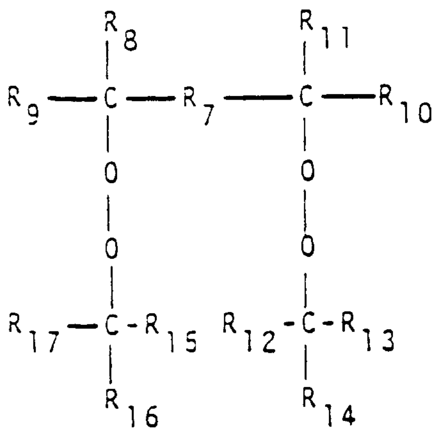

(II)

dans laquelle :

- $R_7$ représente alkylène en $C_{1-6}$, alcénylène en $C_{2-6}$, alcynylène en $C_{2-6}$ ou phénylène ;
- $R_8$, $R_9$, $R_{10}$ et $R_{11}$, identiques ou différents les uns des autres, représentent des groupes alkyle en $C_{1-3}$ ;
- $R_{13}$ et $R_{15}$, identiques ou différents l'un de l'autre, représentent des groupes alkyle en $C_{2-4}$ ;
- $R_{12}$, $R_{14}$, $R_{16}$ et $R_{17}$, identiques ou différents les uns des autres, représentent des groupes alkyle en $C_{1-4}$ ;

à la condition que, lorsque $R_7$ représente phénylène, $R_8$ et $R_{11}$, identiques ou différents l'un de l'autre, représentent des groupes alkyle en $C_{2-4}$.

**2.** Mélanges selon la revendication 1, dans lesquels le peroxyde est choisi parmi le bis(1,1-diméthylpropyl)peroxyde, le bis(1,1-diéthylpropyl)peroxyde, le bis(1-éthyl-1-méthylpropyl)peroxyde, le 1,1-diéthylpropyl, 1-éthyl-1-méthylpropyl)peroxyde, le 2,5-diméthyl-2,5-di-tert.-amylperoxy-hexane et leurs mélanges.

**3.** Mélanges selon l'une des revendications 1 et 2, contenant en outre, de préférence dans des quantités de 0,1 à 1 partie en poids, du benzothiazole ou des dérivés de celui-ci, en particulier le disulfure de mercaptobenzothiazole.

**4.** Mélanges selon l'une des revendications 1 à 3, contenant, de préférence dans des quantités de 0,1 à 2 parties en poids, du N-phényl maléimide.

**5.** Bis(1,1-diéthylpropyl)peroxyde.

**6.** Bis(1-éthyl-1-méthylpropyl)peroxyde.

**7.** 1,1-Diéthylpropyl, 1-éthyl-1-méthylpropylperoxyde.